(19) Europäisches Patentamt
European Patent Office
Office européen des brevets

(11) Publication number: **0 184 258 B1**

# (12) EUROPEAN PATENT SPECIFICATION

(45) Date of publication of patent specification: **06.05.92**

(51) Int. Cl.$^5$: **C07D 401/06**, C07D 401/14, C07D 405/04, C07D 405/14, A61K 31/505

(21) Application number: **85201938.9**

(22) Date of filing: **22.11.85**

The file contains technical information submitted after the application was filed and not included in this specification

## (54) Novel derivatives of hydroxy- or amino-substituted (piperidinylalkyl)quinazolines.

(30) Priority: **05.12.84 US 678422**

(43) Date of publication of application:
**11.06.86 Bulletin 86/24**

(45) Publication of the grant of the patent:
**06.05.92 Bulletin 92/19**

(84) Designated Contracting States:
**AT BE CH DE FR GB IT LI LU NL SE**

(56) References cited:
**EP-A- 0 013 612**

(73) Proprietor: **JANSSEN PHARMACEUTICA N.V.**
**Turnhoutsebaan 30**
**B-2340 Beerse(BE)**

(72) Inventor: **Vandenberk, Jan**
**Kempenlaan 15**
**B-2340 Beerse(BE)**
Inventor: **Kennis, Ludo Edmond**
**Guido Gezellestraat 50**
**B-2300 Turnhout(BE)**
Inventor: **Mertens, Josephus Carolus**
**Staatsbaan 35/1**
**B-2360 Oud-Turnhout(BE)**
Inventor: **Van Heertum, Albertus Hendricus Maria T.**
**Albertstraat 10**
**B-2350 Vosselaar(BE)**

EP 0 184 258 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

(Piperidinylalkyl)-quinazoline derivatives having potent serotonin-antagonistic properties have been described in U.S. Patent No. 4,335,127. The compounds of the present invention differ from the hereinabove-mentioned prior-art compounds by their substitution on the quinazoline moiety and by their increased serotonin-antagonistic properties.

The present invention is concerned with quinazoline derivatives which are structurally represented by the formula

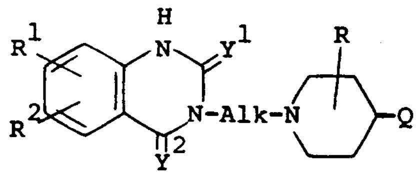 (I)

and the pharmaceutically acceptable acid addition salts thereof, wherein

R is hydrogen or $C_{1-6}$ alkyl;

$R^1$ is amino, mono- and di($C_{1-6}$ alkyl)amino, $C_{1-10}$ alkylcarbonylamino or an azido group;

$R^2$ is hydrogen or halo;

$Y^1$ and $Y^2$ are each independently O or S;

Alk is a $C_1$-$C_6$ alkanediyl radical; and

Q is 1H-indol-3-yl or a radical of formula

-X-Ar (a)

wherein Ar is aryl; and X is a bivalent radical selected from the group consisting >C=O; >CHOH; >CH-O-C(=O)-$R_a$; >$CH_2$; >C(O-$C_{1-6}$ alkyl)$_2$;

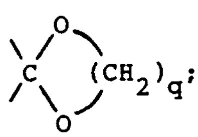

>C=NOH and >C=N-$NH_2$

said $R_a$ being hydrogen or $C_{1-6}$ alkyl and said q being the integer 2 or 3;

wherein aryl as used in the definition of Ar is phenyl optionally substituted with up to three halo-, $C_{1-6}$ alkyl-, $C_{1-6}$ alkyloxy-, trifluoromethyl or amino groups, thienyl or pyridinyl.

In the foregoing definitions the term halo is generic to fluoro, chloro, bromo and iodo; "$C_{1-6}$ alkyl" is meant to include straight and branched saturated hydrocarbon radicals, having from 1 to 6 carbon atoms, such as, for example, methyl, ethyl, 1-methylethyl, 1,1-dimethylethyl, propyl, butyl, pentyl, hexyl; "$C_{1-10}$ alkyl" is meant to include $C_{1-6}$ alkyl radicals, as defined hereinabove, and the higher homologs thereof having from 7 to 10 carbon atoms; and "$C_1$-$C_6$ alkanediyl" is meant to include bivalent straight or branch chained alkanediyl radicals having from 1 to 6 carbon atoms.

Preferred compounds within the scope of formula (I) are those wherein $R^1$ is amino, acetylamino or azido, $R^2$ is hydrogen, R is hydrogen and Q is -CO-Ar.

Particularly preferred compounds within the scope of formula (I) are those wherein R, $R^1$ and $R^2$ are described for the preferred compounds, and wherein Q is -CO-Ar, wherein Ar is halophenyl.

The most preferred compound within the scope of formula (I) is 7-azido-3-[2-[4-(4-fluorobenzoyl)-1-piperidinyl)ethyl]-2,4-(1,3H)-quinazolinedione and their pharmaceutically acceptable acid-addition salts.

The compounds of formula (I) can generally be prepared by reacting an appropriate reactive ester of formula (II) with a piperidine of formula (III).

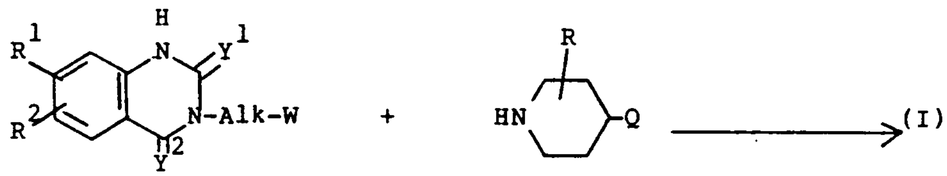

(II) (III)

In (II) and (III) $R^1$, $R^2$, $Y^1$, $Y^2$, Alk, R and Q are as previously defined and W is a reactive ester residue such as, for example, halo, e.g. chloro, bromo or iodo, or a sulfonyloxy radical such as methylsulfonyloxy, 4-methylphenylsulfonyloxy.

The above-mentioned reactions are conveniently conducted in an inert organic solvent such as, for example, an aromatic hydrocarbon, e.g., benzene, methylbenzene, dimethylbenzene; a lower alkanol, e.g., methanol, ethanol, 1-butanol; a ketone, e.g., 2-propanone, 4-methyl-2-pentanone; an ether, e.g., 1,4-dioxane, 1,1'-oxybisethane, tetrahydrofuran; N,N-dimethylformamide (DMF); N,N-dimethylacetamide (DMA); nitrobenzene; 1-methyl-2-pyrrolidinone. The addition of an appropriate base such as, for example, an alkali metal carbonate or hydrogen carbonate, sodium hydride or an organic base such as, for example, N,N-diethylethanamine or N-(1-methylethyl)-2-propanamine may be utilized to pick up the acid which is liberated during the course of the reaction. In some circumstances the addition of an iodide salt, preferably an alkali metal iodide, is appropriate. Somewhat elevated temperatures may enhance the rate of the reaction.

In order to simplify the structural representation of the compounds of formula (I) and of certain intermediates thereof the radical of formula

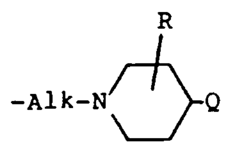

will hereinafter be represented by the symbol D.

The compounds of formula (I) may also be prepared by cyclizing an intermediate of formula (IV) with an amine of formula (V).

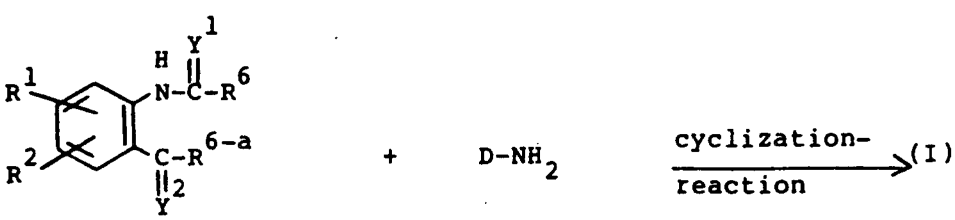


(IV) (V)

In (IV) $R^6$ and $R^{6\text{-}a}$ represent each an appropriate leaving group such as, for example, $C_{1-6}$ alkyloxy, amino and mono- and di ($C_{1-6}$ alkyl)-amino.
In the reaction of (IV) with (V) the intermediately formed amide of formula

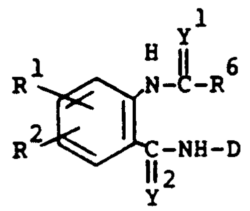

(VI)

may be isolated before the start of the cyclization-reaction.

Additionally, the compounds of formula (I) may be prepared by cyclizing an isocyanate or isothiocyanate of formula (VII) with an amine of formula (V).

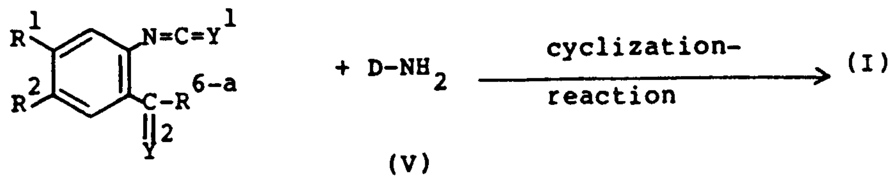


(VII) (V)

Said cyclization-reactions are conveniently conducted by heating the reactants together, optionally in a suitable reaction-inert solvent having a relatively high boiling point such as aliphatic and aromatic hydrocarbons, e.g. petroleumether, dimethylbenzene, an ether, e.g. tetrahydrofuran, water. The presence of a suitable base, e.g. an alkalimetal or earth alkaline metal hydroxide, such as potassium hydroxide, may enhance the rate of the reaction.

The compounds of formula (I) wherein $R^1$ is amino, said compounds being represented by the formula (I-a), may also be derived from the corresponding nitro-substituted quinazolines of formula (VIII) following art-known nitro-to-amine reduction procedures.

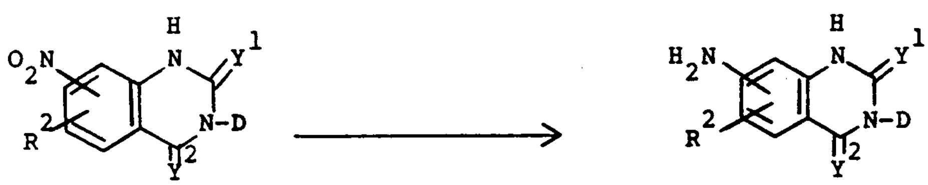

(VIII) (I-a)

A suitable nitro-to-amine reducing procedure is, for example, catalytic hydrogenation in a relatively polar solvent such as, for example, an alcohol, e.g. methanol or ethanol, in the presence of an appropriate catalyst, e.g. platinum-on-charcoal. In some cases it may be useful to add an appropriate catalyst poison, e.g. thiophene.

The compounds of formula (I) may also be converted into each other following art-known functional group transformation procedures.

For example, the compounds of formula (I) wherein $R^1$ is amino may be converted into compounds of formula (I) wherein $R^1$ is $C_{1-6}$ alkylcarbonylamino by reacting the former compounds with a suitable acylating agent, e.g. an acylhalide or an acid anhydride; the compounds of formula (I) wherein $R^1$ is an amino-group may be converted into compounds of formula (I) wherein $R^1$ is an azido-group by converting the amino-group into a diazonium group with nitrous acid or an appropriate alkalimetal or earth alkaline metal thereof and subsequently converting the said diazonium group into an azide group with sodium azide or any other suitable alkalimetal or earth alkaline metal azide; the compounds of formula (I) wherein $R^2$ is hydrogen may be converted into compounds of formula (I) wherein $R^2$ is halo following art-known

procedures for halogenating aromatic rings; compounds of formula (I) wherein Q is -CO-Ar may be converted into compounds of formula (I) wherein Q is -CHOH-Ar following art-known carbonyl-to-alcohol reducing procedures, e.g. by using sodiumborohydride as reductans; and compounds of formula (I) wherein X in X-Ar is $>C\text{-}(O\text{-}C_{1-6}\ \text{alkyl})_2$ or

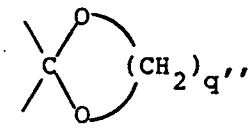

may be converted into compounds of formula (I) wherein Q is -CO-Ar by reacting the former with a suitable acid.

The intermediates and starting materials in the foregoing preparations may be prepared according to art-known methodologies for preparing said or similar chemical compounds, as described, for example in US Patent No. 4,335,127.

The compounds of formula (I) have basic properties and, consequently, they may be converted to their therapeutically active non-toxic acid addition salt forms by treatment with appropriate acids, such as, for example, inorganic acids, such as hydrohalic acid, e.g. hydrochloric, hydrobromic, and sulfuric acid, nitric acid, phosphoric acid; or organic acids, such as, for example, acetic, propanoic, hydroxyacetic, 2-hydroxy-propanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, benzoic, 2-hydroxybenzoic, 3-phenyl-2-propenoic, α-hydroxybenzeneacetic, 4-amino-2-hydroxybenzoic acid. Conversely the salt form can be converted by treatment with alkali into the free base form.

The compounds of formula (I) and their pharmaceutically active acid addition salts have useful pharmacological properties. They are very potent serotonin-antagonists and as such they can be used in the treatment of a variety of diseases in which serotonin release is of predominant importance.

The potency of the subject compounds as serotonin-antagonists is clearly evidenced by the results obtained in the following tests wherein the antagonistic activity of the compounds (I) on the effect of serotonin is examined.

Test 1: Antagonistic activity on the effect of serotonin on the caudal artery of the rat

Caudal arteries from fasted male rats (210-235 g) were used in the test. Two helical strips having a length of 5-6 cm and a width of 2 mm were obtained from each artery and mounted vertically in a 100 ml organ bath containing an oxygenated Krebs-Henseleit solution. Submaximal contractions of the arterial strips were produced by adding single doses of serotonin (40 ng/ml) to the organ bath for 2 minutes with each time an interval of 10 minutes. The amplitude of the contraction is measured before and 5 minutes after adding the drug. After washing out, the agonist was added again three times in order to see whether the contraction is restored and normalized.

The first column of table 1 shows the $ED_{50}$-values in ng/ml for a number of compounds of formula (I) in the above test. In this connection the $ED_{50}$-values are the minimal concentrations of the concerned drugs which reduce the amplitude of the contraction to at least 50 % of its normal value.

Test 2: Effects in gastric lesion tests

a. Lesions induced by compound 48/80:

Compound 48/80 (a mixture of oligomers obtained by condensation of 4-methoxy-N-methylbenzenethanamine and formaldehyde) is a potent releaser of vasoactive amines from endogenous stores such as, for example, histamine and serotonin. Rats injected with compound 48/80 exhibit consistent changes of blood flow in different vascular beds: cyanosis of the ears and the extremities are prominent within five minutes after injection of the compound; the rats die from shock within 30 minutes. The shock, followed by dead, can be avoided if the rats are pretreated with a classical HI antagonist. However the stimulatory effects on gastric secretion are not suppressed so that rats treated with compound 48/80 and protected from shock by an HI antagonist may exhibit all signs of intensive gastric gland activity: gross autopsy shows distended stomachs with abnormal contents and rough bright red patches all over the mucosa, corresponding to areas of disintegrated glands. A number of known serotonin antagonists such as, for examples, methysergide, cyproheptadine, cinanserin, mianserin, pipamperone, spiperone, pizotifen and metergoline, prevent completely the cyanosis of ears and extremities as well as the lesions in the glandular area of the stomach and the abnormal gastric distension.

b. Method:

Male rats of a Wistar inbred strain, weighing 220-250 g, were starved overnight, water being available ad libitum. The test compounds were administered orally as a solution or as a suspension in aqueous medium. A control rat and a "blank" rat receive the test compound. One hour later 5-[4-(diphenylmethyl)-1-piperazinyl-methyl]-1-methyl-1H-benzimidazole-2-methanol is administered subcutaneously to all rats at the dose of 2.5 mg/kg. Two hours after the oral administration of the test compound the compound 48/80 (freshly dissolved in water at a concentration of 0.25 mg/ml) was injected intravenously into all rats (dose: 1 mg/kg) except the "blank" rats. Five minutes after the injection the intensity of purple-blue coloration (cyanosis) of the extremities was scored as 0 (absent), + (moderate) or + + (intense). Four hours after the intravenous injection of compound 48/80 the rats were decapitated and the stomachs were removed. Subsequently the stomachs were inspected for distension and contents (blood, fluid, food) and thoroughly rinsed. The macroscopic lesions were scored from 0 to + + +, 0 corresponding to complete absence of visible lesions and the highest score corresponding to reddish rough patches covering more than half the glandular area.

The second column of table 1 shows for a number of compounds of formula (I) the doses (in mg/kg body weight) at which the distension of the stomach as well as the lesions in the glandular area of the stomach are completely absent in 50 % of the test rats ($ED_{50}$-values). The compounds listed in table 1 are not given

for the purpose of limiting the invention thereto but only to exemplify the useful pharmacological activities of all the compounds within the scope of formula (I).

Table 1

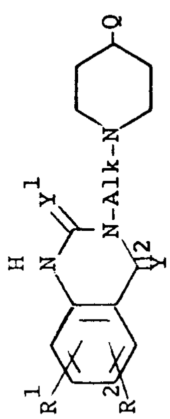

| No. | $R^1$ | $R^2$ | $Y^1$ | $Y^2$ | Alk | Q | Caudal artery ng/ml | Gastric lesion $ED_{50}$ in mg/kg |
|---|---|---|---|---|---|---|---|---|
| 1 | 7-$NH_2$ | H | O | O | $(CH_2)_4$ | 4-F-$C_6H_4$-CO- | 0.32 | 0.04 |
| 2 | 7-$NH_2$ | H | S | O | $(CH_2)_2$ | 4-F-$C_6H_4$-CO- | 0.18 | 0.005 |
| 4 | 7-$NH_2$ | H | S | O | $(CH_2)_4$ | 4-F-$C_6H_4$-CO- | $<$ 0.63 | 0.63 |
| 5 | 7-$NH_2$ | H | O | O | $(CH_2)_2$ | 4-F-$C_6H_4$-CO- | $<$ 0.63 | 0.04 |
| 9 | 7-NHAc | H | O | O | $(CH_2)_4$ | 4-F-$C_6H_4$-CO- | 0.32 | 0.08 |
| 10 | 7-NHAc | H | S | O | $(CH_2)_2$ | 4-F-$C_6H_4$-CO- | 0.32 | 0.01 |
| 13 | 7-NHAc | H | O | O | $(CH_2)_2$ | 4-F-$C_6H_4$-CO- | 0.37 | 0.04 |

The compounds of formula (I) and their pharmaceutically acceptable acid addition salts are selective serotonin $S_2$-antagonists which bind with high affinity to $S_2$ (or 5-$HT_2$) receptors, directly antagonize serotonin-induced platelet activation and blood vessel contraction and prevent the functional consequences of serotonergic overstimulation such as vascular congestion and subsequent organ deficiency (e.g. gastric lesions). Consequently the compounds of the present invention can be used in the treatment of a broad spectrum of diseases according to the vascular bed in which excessive serotonin release occurs, primarily in hypertension and carcinoid syndrome but also in Raynaud's disease, gastrointestinal ulcus , scleroderma, hemorroids, irritable bowel syndrom and the like diseases whenever platelet activation with a thrombotic tendency or an excessive serotonin release from enterochromaffin cells is present.

In view of their useful selective serotonin $S_2$-antagonistic properties, the subject compounds may be formulated into various pharmaceutical forms for administration purposes. To prepare the pharmaceutical composition of this invention, an effective serotonin antagonistic amount of the particular compound, in base or acid-addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, preferably, for administration orally, rectally or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents may be employed. Acid addition salts of (I), due to their increased water solubility over the corresponding base form, are obviously more suitable in the preparation of aqueous compositions. It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and segregated multiples thereof.

Although the amount of the active ingredient to be administered may vary within rather wide limits depending on the particular circumstances, such as the nature and the severity of the disease, doses of from about 0.04 to about 4 mg of active ingredient per kg of body weight, and particularly from about 0.1 to about 2 mg per kg of body weight, administered once or repeatedly, are in general satisfactory.

The following formulations exemplify typical serotonin antagonistic pharmaceutical compositions in dosage unit form suitable for systemic administration to animal and human subjects in accordance with the present invention.

"Active ingredient" (A.I.) as used throughout these formulations relates to a compound of formula (I) or a pharmaceutically acceptable acid-addition salt thereof.

## ORAL DROPS

500 Grams of the A.I. was dissolved in 0.5 liters of 2-hydroxypropanoic acid and 1.5 liters of the polyethylene glycol at 60-80° C. After cooling to 30-40° C there were added 35 liters of polyethylene glycol and the mixture was stirred well. Then there was added a solution of 1750 grams of sodium saccharin in 2.5 liters of purified water end while stirring there were added 2.5 liters of cocoa flavor and polyethylene glycol q.s. to a volume of 50 liters, providing an oral drop solution comprising 10 milligrams of the A.I. per milliliter. The resulting solution was filled into suitable containers.

## ORAL SOLUTION

9 Grams of methyl 4-hydroxybenzoate and 1 gram of propyl 4-hydroxybenzoate were dissolved in 4 liters of boiling purified water. In 3 liters of this solution were dissolved first 10 grams of 2,3-dihydroxybutanedioic acid and thereafter 20 grams of the A.I. The latter solution was combined with the remaining part of the former solution and 12 liters 1,2,3-propanetriol and 3 liters of sorbitol 70% solution were added thereto. 40 Grams of sodium saccharin were dissolved in 0.5 liters of water and 2 milliliters of raspberry and 2 milliliters of gooseberry essence were added. The latter solution was combined with the former, water was added q.s. to a volume of 20 liters providing an oral solution comprising 20 milligrams of the active ingredient per teaspoonful (5 milliliters). The resulting solution was filled in suitable containers.

CAPSULES

20 Grams of the A.I., 6 grams sodium lauryl sulfate, 56 grams starch, 56 grams lactose, 0.8 grams colloidal silicon dioxide, and 1.2 grams magnesium stearate were vigorously stirred together. The resulting mixture was subsequently filled into 1000 suitable hardened gelating capsules, comprising each 20 milligrams of the active ingredient.

FILM-COATED TABLETS

Preparation of tablet core : A mixture of 100 grams of the A.I., 570 grams lactose and 200 grams starch was mixed well and thereafter humidified with a solution of 5 grams sodium dodecyl sulfate and 10 grams polyvinylpyrrolidone in about 200 milliliters of water. The wet powder mixture was sieved, dried and sieved again. Then there was added 100 grams microcrystalline cellulose and 15 grams hydrogenated vegetable oil. The whole was mixed well and compressed into tablets, giving 10.000 tablets, each containing 10 milligrams of the active ingredient. Coating : To A solution of 10 grams methyl cellulose in 75 milliliters of denaturated ethanol there was added a solution of 5 grams of ethyl cellulose in 150 milliliters of dichloromethane. Then there were added 75 milliliters of dichloromethane and 2.5 milliliters 1,2,3-propanetriol. 10 Grams of polyethylene glycol was molten and dissolved in 75 milliliters of dichloromethane. The latter solution was added to the former and then there were added 2.5 grams of magnesium octadecanoate, 5 grams of polyvinylpyrrolidone and 30 milliliters of concentrated colour suspension (Opaspray K-1-2109) and the whole was homogenated.
The tablet cores were coated with the thus obtained mixture in a coating apparatus.

INJECTABLE SOLUTIONS

1.8 Grams methyl 4-hydroxybenzoate and 0.2 grams propyl 4-hydroxy-benzoate were dissolved in about 0.5 liters of boiling water for injection. After cooling to about 50° C there were added while stirring 4 grams lactic acid, 0.05 propylene glycol and 4 grams of the A.I.. The solution was cooled to room temperature and supplemented with water for injection q.s. ad 1 liter volume, giving a solution of 4 milligrams A.I. per milliliters. The solution was sterilized by filtration (U.S.P. XVII p. 811) and filled in sterile containers.

SUPPOSITORIES

3 Grams A.I. was dissolved in a solution of 3 grams 2,3-dihydroxy-butanedioic acid in 25 milliliters polyethylene glycol 400. 12 Grams surfactant and triglycerides q.s. ad 300 grams were melted together. The latter mixture was mixed well with the former solution. The thus obtained mixture was poured onto moulds at a temperature of 37-38° C to form 100 suppositories each containing 30 milligrams of the active ingredient.

In the following examples, unless otherwise stated, all parts are by weight.

EXPERIMENTAL PART

A. Preparation of intermediates:

Example 1

A mixture of 74.7 parts of 1-acetyl-4-[(4-fluorophenyl)carbonyl] piperidine, 46.5 parts of 1,2-ethanediol, 3 parts of 4-ethylbenzenesulfonic acid and 810 parts of benzene was stirred and refluxed for 108 hours with water-separator. The reaction mixture was cooled and washed successively with a mixture of 250 parts of water and 22.5 parts of ammonium hydroxide, and with 250 parts of water. The organic phase was separated, dried, filtered and evaporated. The residue was purified by column chromatography over silica gel using a mixture of trichloromethane and 2-propanone (50:50 by volume) as eluent. The pure fractions were collected and the eluent was evaporated, yielding 50 parts (56.8 %) of 1-acetyl-4-[(2-(4-fluorophenyl)-1,3-dioxolan-2-y]piperidine as a residue (interm. 1).

A mixture of 5 parts of 1-acetyl-4-[2-(4-fluorophenyl)-1,3-dioxolan-2-yl]piperidine and 100 parts of a sodium hydroxide solution 10 % was stirred and refluxed overnight. The reaction mixture was cooled and the product was extracted with trichloromethane. The extract was dried, filtered and evaporated. The solid residue was stirred in 2,2'-oxybispropane. The product was filtered off and dried in vacuo at 40 ° C, yielding 3.5 parts (82 %) of 4-[2-(4-fluorophenyl)-1,3-dioxolan-2-yl]piperidine (2).

Example 2

A mixture of 5 parts of 4-chlorobutanenitrile, 10 parts of (4-fluorophenyl)(4-piperidinyl)methanone, 10 parts of sodium carbonate and 200 parts of 4-methyl-2-pentanone was stirred and refluxed overnight. The reaction mixture was filtered and the filtrate was evaporated. The oily residue was crystallized from 2,2'-oxybispropane. The product was filtered off and recrystallized from 2,2'-oxybispropane, yielding 6.1 parts of 4-(4-fluorobenzoyl)-1-piperidinebutanenitrile; mp. 100 ° C (3).

Following the same procedure and using equivalent amounts of the appropriate starting materials there were also prepared:
4-(4-fluorobenzoyl)-1-piperidineacetonitrile (4);
4-(1H-indol-3-yl)-1-piperidineacetonitrile (5);
4-[2-(4-fluorophenyl)-1,3-dioxolan-2-yl]-1-piperidineacetonitrile (6); and
4-[2-(4-fluorophenyl)-1,3-dioxolan-2-yl]-1-piperidinebutanenitrile (7).

Example 3

A mixture of 28 parts of 4-[2-(4-fluorophenyl)-1,3-dioxolan-2-yl]-1-piperidinebutanenitrile and 200 parts of methanol saturated with ammonia was hydrogenated at normal pressure and at room temperature with 5 parts of Raney nickel catalyst. After the calculated amount of hydrogen was taken up, the catalyst was filtered off over Hyflo and the filtrate was evaporated, yielding 25 parts (95 %) of 4-[2-(4-fluorophenyl)-1,3-dioxolan-2yl]-1-piperidinebutanamine as a residue (3).

In a similar manner there were also prepared:
[1-(2-aminoethyl)-4-piperidinyl](4-fluorophenyl)methanone as a residue; (9)
4-(1H-indol-3-yl)-1-piperidineethanamine; (10)
[1-(4-aminobutyl)-4-piperidinyl](4-fluorophenyl)methanone as a residue (11); and
4-[2-(4-fluorophenyl)-1,3-dioxolan-2-yl]-1-piperidineethanamine as a residue (12).

Example 4

To a stirred solutuion of 28 parts of [1-(4-aminobutyl)-4-piperidinyl](4-fluorophenyl)methanone in 135 parts of tetrahydrofuran was added dropwise during a 10 minutes-period a solution of 24 parts of methyl 2-isothiocyanato-4-nitrobenzoate in 180 parts of tetrahydrofuran (slightly exothermic reaction). Upon completion, stirring was continued overnight at room temperature. The solid was filtered off and crystallized from ethanol while stirring and cooling to 40 ° C. The product was filtered off, washed with ethanol and dried in vacuo at 80 ° C, yielding 30 parts (62 %) of 3-[4-[4-(4-fluorobenzoyl)-1-piperidinyl]butyl]-2,3-dihydro-7-nitro-2-thioxo-4(1H)-quinazolinone; mp. 225 ° C (13).

Following the same procedure and using equivalent amounts of the appropriate starting materials there were also prepared: 3-[2-[4-(4-fluorobenzoyl)-1-piperidinyl)ethyl]-2,3-dihydro-7-nitro-2-thioxo-4(1H)-quinazolinone (14); and 3-[2-[4-[2-(4-fluorophenyl)-1,3-dioxolan-2-yl]-1-piperidinyl)ethyl]-2,3-dihydro-7-nitro-2-thioxo-4(1H)-quinazolinone: mp. 217.8 ° C (15).

Example 5

To a stirred mixture of 7 parts of 3-[2-[2-[4-(4-fluorobenzoyl)-1-piperidinyl]ethyl]-2,3-dihydro-7-nitro-2-thioxo-4-(1H)-quinazolinone, 120 parts of a solution of potassium hydroxide in ethanol 5 % and 10 parts of water were added dropwise 100 parts of a hydrogen peroxide solution 3 % during a 10 minutes period. Upon completion, stirring was continued for 2.50 hours at room temperature. Then there were added 100 parts of water and the whole was neutralized with acetic acid. The precipitated product was filtered off and crystallized from a mixture of N,N-dimethylacetamide and water, yielding 4.8 parts of 3-[2-[4-(4-fluorobenzoyl)-1-piperidinyl]ethyl]-7-nitro-2,4(1H, 3H)-quinazolinedione; mp. 238.1 - 240.1 ° C (16).

Example 6

To a stirred mixture of 36 parts of 3-[4-[4-(4-fluorobenzoyl)-1-piperidinyl]butyl]-2,3-dihydro-7-nitro-2-thioxo-4(1H)-quinazolinone, 700 parts of a potassium hydroxide solution in ethanol 5 % and 150 parts of water were added 500 parts of a hydrogen peroxide solution 3 % at room temperature. The whole was stirred overnight at room temperature. The mixture was neutralized with acetic acid. The product was filtered off, washed with water and 2-propanol and stirred in 2,2'-oxybispropane and thrichloromethane. The product was filtered off and dried, yielding 31 parts (88 %) of 3-[4-[4-(4-fluorobenzoyl)-1-piperidinyl]butyl]-7-nitro-2,4(1H,3H)-quinazolinedione; mp. 188° C (17).

Example 7

To a stirred mixture of 22.5 parts of 3-(2-chloroethyl)-2,4(1H,3H)-quinazolinedione and 368 parts of sulfuric acid (d = 1.84) were added dropwise 8.1 parts of nitric acid (d = 1.5) while the temperature was kept at room temperature by cooling in an ice-water bath. Upon completion, stirring at room temperature was continued for 3 hours. The reaction mixture was poured onto crushed ice. The precipitated product was washed three times with water and crystallized from methanol. Upon cooling, the product was filtered off and dried, yielding 25 parts (100 %) of 3-(2-chloroethyl)-6-nitro-2,4(1H,3H)-quinazolinedione; mp. 251.2° C (18).

Example 8

A mixture of 16.2 parts of 3-(2-chloroethyl)-6-nitro-2,4(1H,3H)-quinazolinedione, 15 parts of 4-[2-(4-fluorophenyl)-1,3-dioxolan-2-yl]piperidine, 10 parts of sodium hydrogen carbonate, 0.1 parts of potassium iodide and 135 parts of N,N-dimethylformamide was stirred and heated overnight at 100-120° C. The reaction mixture was cooled and poured onto water. Stirring was continued till complete precipitation. The precipitate was filtered off and dissolved in trichloromethane. The thus formed emulsion was dried, filtered and evaporated. The residue was crystallized from acetonitrile, yielding 18.6 parts (64 %) of 3-[2-[4-[2-(4-fluorophenyl)-1,3-dioxolan-2-yl]-1-piperidinyl]ethyl]-6-nitro-2,4(1H,3H)-quinazolinedione; mp. 250.9° C (19).

Following the same procedure and using equivalent amounts of the appropriate starting materials there were also prepared:

3-[2-[4-(4-fluorobenzoyl)-1-piperidinyl]ethyl]-6-nitro-2,4(1H,3H)-quinazolinedione (Z)-2-butenedioate(1:1)-.hemihydrate; mp. 213.7° C (20); and

3-[2-[4-(1H-indol-3-yl)-1-piperidinyl]ethyl]-6-nitro-2,4(1H,3H)-quinazolinedione; mp. 228.2° C (21).

B. Preparation of Final Compounds

Example 9

A mixture of 30 parts of 3-[4-[4-(4-fluorobenzoyl)-1-piperidinyl]-butyl]-7-nitro-2,4(1H,3H)-quinazolinedione, 500 parts of acetic acid and 2 parts of a solution of thiophene in methanol 4 % was hydrogenated at normal pressure and at room temperature with 5 parts of palladium-on-charcoal catalyst 10 %. After the calculated amount of hydrogen was taken up, the catalyst was filtered off over diatomaceous earth and the filtrate was evaporated. The residue was stirred in 80 parts of 2-propanol, 300 parts of water and 100 parts of ammonium hydroxide. The aqueous phase was decanted and the residue was crystallized from acetonitrile. The product was filtered off and dried, yielding 27 parts (96 %) of 7-amino-3-[4-[4-(4-fluorobenzoyl)-1-piperidinyl]butyl]-2,4(1H,3H)-quinazolinedione; mp. 213.0° C (1).

In a similar manner there were also prepared:

7-amino-3-[2-[4-(4-fluorobenzoyl)-1-piperidinyl]ethyl]-2,3-dihydro-2-thioxo-4(1H)-quinazolinone; mp. 262.6° C (2);

7-amino-3-[2-[4-[2-(4-fluorophenyl)-1,3-dioxolan-2-yl]-1-piperidinyl]ethyl]-2,3-dihydro-2-thioxo-4(1H)-quinazolinone acetate(1:1) as a residue (3);

7-amino-3-[4-[4-(4-fluorobenzoyl)-1-piperidinyl]butyl]-2,3-dihydro-2-thioxo-4(1H)-quinazolinone; mp. 194.8° C (4);

7-amino-3-[2-[4-(4-fluorobenzoyl)-1-piperidinyl]ethyl]-2,4(1H,3H)-quinazolinedione; mp. 266.3° C (5);

6-amino-3-[2-[4-(4-fluorobenzoyl)-1-piperidinyl]ethyl]-2,4(1H,3H)-quinazolinedione; mp. 258.6° C (6); and

6-amino-3-[2-[4-(1H-indol-3-yl)-1-piperidinyl]ethyl]-2,4[1H,3H)-quinazolinedione; mp. 277.0° C (7).

Example 10

A mixture of 1.2 parts of 3-[2-[4-[4-fluorobenzoyl)-1-piperidinyl)ethyl]-6-nitro-2,4(1H,3H)-quinazolinedione, 0.5 parts of a solution of thiophene in methanol 4 % and 100 parts of acetic acid was hydrogenated at normal pressure and at room temperature with 1 part of platinum-on-charcoal catalyst 5 %. After the calculated amount of hydrogen was taken up, the catalyst was filtered off and 10 parts of acetic acid anhydride were added to the filtrate. The whole was stirred and refluxed for 3 hours. After evaporation, water was added to the residue and the whole was treated with ammonia. The precipitated product was filtered off and converted into the (Z)-2-butenedioate salt in ethanol. From the latter the salt was crystallized at -5°C. The product was filtered off and dried, yielding 0.4 parts (32 %) of N-[3-[2-[4-(4-fluorobenzoyl)-1-piperidinyl]ethyl]-1,2,3,4-tetrahydro-2,4-dioxo-6-quinazolinyl]-acetamide (Z)-2-butenedioate(1:1)-.monohydrate; mp. 170.7°C (8).

Example 11

A mixture of 5.8 parts of 7-amino-3-[4-[4-(4-fluorobenzoyl)-1-piperidinyl]butyl]-2,4(1H,3H)-quinazolinedione, 100 parts of acetic acid and 2.5 parts of acetic acid anhydride was stirred at reflux temperature. The reaction mixture was evaporated. The residue was stirred in water and ammonium hydroxide. The product was filtered off, washed with water and boiled in ethanol. After cooling, the product was filtered off, washed with 2,2'-oxybispropane and dried, yielding 6.3 parts (100 %) of N-[3-[4-[4-(4-fluorobenzoyl)-1-piperidinyl]butyl]-1,2,3,4-tetrahydro-2,4-dioxo-7-quinazolinyl]acetamide; mp. 273.1°C (9).
In a similar manner there were also prepared:
N-[3-[2-[4-(4-fluorobenzoyl)-1-piperidinyl]ethyl]-1,2,3,4-tetrahydro-4-oxo-2-thioxo-7-quinazolinyl]acetamide; mp. 246.9°C (10);
N-[3-[2-[4-[2-(4-fluorophenyl)-1,3-dioxolan-2-yl]-1-piperidinyl]ethyl]-1,2,3,4-tetrahydro-4-oxo-2-thioxo-7-quinazolinyl]acetamide monohydrate; mp. 240.7°C (11).
N-[3-[4-[4-(4-fluorobenzoyl)-1-piperidinyl]butyl]-1,2,3,4-tetrahydro-4-oxo-2-thioxo-7-quinazolinyl]acetamide; mp. 246.8°C (12); and
N-[3-[2-[4-(4-fluorobenzoyl)-1-piperidinyl]ethyl]-1,2,3,4-tetrahydro-2,4-dioxo-7-quinazolinyl]-acetamide; mp. 284.4°C (13).

Example 12

To a stirred and cooled (5°C) solution of 0.205 parts of 7-amino-3-[2-[4-(4-fluorobenzoyl)-1-piperidinyl]-ethyl]-2,4(1H,3H)-quinazolinedione in 50 parts of an acetic acid solution 1N was added a solution of 0.1725 parts of sodium nitrite in 2 parts of water. The whole was stirred for 20 minutes at 5°C. A solution of 0.1625 parts of sodium azide in 2 parts of water was added and stirring was continued for 20 minutes at 5°C in vacuo. 5.5 Parts of a sodium hydroxide solution 10N were added whereupon the product solidified. The product was filtered off and crystallized from acetonitrile. The product was filtered off and dried, yielding 0.120 parts (55 %) of 7-azido-3-[2-[4-(4-fluorobenzoyl)-1-piperidinyl]ethyl]-2,4(1H, 3H)quinazolinedione; mp. 193.7°C (14).
In a similar manner there was also prepared:
6-azido-3-[2-[4-(4-fluorobenzoyl)-1-piperidinyl]ethyl]-2,4(1H,3H)-quinazolinedione; mp. 245.8°C (15).

Example 13

To a stirred solution of 0.205 parts of 7-amino-3-[2-[4-(4-fluorobenzoyl)-1-piperidinyl]ethyl]-2,4(1H,3H)-quinazolinedione in 160 parts of methanol were added 150 parts of a sodium acetate solution 1M (pH = 5.6). The whole was filtered and to the filtrate was added first a solution of 0.150 parts of sodium iodide in 10 parts of water and a solution of 0.282 parts of N-chloro-4-methylbenzenesulfonamide, sodium salt trihydrate in 10 ports of water. After stirring for 10 minutes at room temperature, a solution of 0.380 parts of sodium disulfurite in 10 parts of water was added. The pH of the mixture was adjusted to 9-10 with a sodium hydroxide solution 10N. The product was extracted three times with 65 parts of dichloromethane and three times with 75 parts of trichloromethane. The combined organic layers were dried, filtered and evaporated. The residue was taken up in 16 parts of acetonitrile. The whole was evaporated to dry, yielding 0.220 parts (82 %) of 7-amino-3-[2-[4-(4-fluorobenzoyl)-1-piperidinyl]ethyl]-8-iodo-2,4(1H, 3H)-quinazolinedione; mp. 209.1°C (16).

Example 14

A mixture of 4.85 parts of 7-amino-3-[2-[4-(4-fluorobenzoyl)-1-piperidinyl]ethyl]-2,4(1H,3H)-quinazolinedione dihydrochloride, 4 parts of potassium acetate, 300 parts of 2-methoxyethanol, 1 part of a solution of thiophene in methanol 4% and 4 parts of poly(oxymethylene) was hydrogenated at normal pressure and at 60 °C with 2 parts of palladium-on-charcoal catalyst 5%. After the calculated amount of hydrogen was taken up (about 8 days), the reaction mixture was diluted with 270 parts of N,N-dimethylacetamide and heated to 100 °C. The catalyst was filtered off over diatomaceous earth. The filtrate was stirred and cooled and 100 parts of water were added. The precipitated product was filtered off, washed with water and stirred in 2-propanone. The product was filtered off and dried, yielding 2.3 parts (52%) of 7-(dimethylamino)-3-[2-[4-(4-fluorobenzoyl)-1-piperidinyl]ethyl]-2,4(1H,3H)-quinazolinedione; mp. 300 °C (17).

## Claims

**1.** A chemical compound having the formula

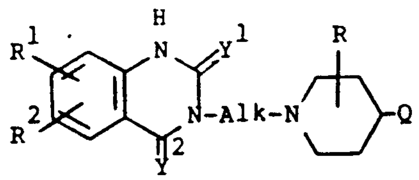

(I)

and a pharmaceutically acceptable acid addition salt thereof, wherein
R is hydrogen or $C_{1-6}$ alkyl;
$R^1$ is amino, mono- and di($C_{1-6}$alkyl)amino, $C_{1-10}$ alkyl carbonylamino or an azido group;
$R^2$ is hydrogen or halo;
$Y^1$ and $Y^2$ are each independently O or S;
Alk is a $C_1$-$C_6$ alkanediyl radical; and
Q is 1 H-indol-3-yl or a radical of formula

-X-Ar (a)

wherein Ar is aryl; and X is a bivalent radical selected from the group consisting of >C=O; >CHOH; >CH-O-C(=O)-$R_a$;
>$CH_2$; >C(O-$C_{1-6}$ alkyl)$_2$;

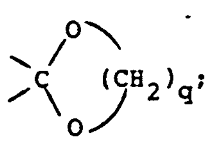

>C=NOH and >C=N-$NH_2$
said $R_a$ being hydrogen or $C_{1-6}$ alkyl and said q being the integer 2 or 3;
wherein aryl as used in the definition of Ar is phenyl optionally substituted with up to three halo-, $C_{1-6}$ alkyl-, $C_{1-6}$ alkyloxy-, trifluoromethyl or amino groups, thienyl or pyridinyl.

**2.** A chemical compound according to claim 1 wherein $R^1$ is amino, acetylamino or azido, $R^2$ and R are both hydrogen and Q is -CO-Ar.

**3.** A chemical compound according to claim 1 wherein the compound is 7-amino-3-[4-(4-fluorobenzoyl)-1-piperidinyl]butyl-2,4(1H,3H)-quinazolinedione.

**4.** A chemical compound according to claim 1 wherein the compound is 7-azido-3-[2-[4-(4-fluorobenzoyl)-1-piperidinyl)ethyl-2,4-(1,3H)-quinazolinedione.

**5.** A pharmaceutical composition comprising a suitable pharmaceutical carrier and as an active ingredient a therapeutically effective amount of a compound as claimed in any one of claims 1 to 4.

**6.** A serotonin-antagonistic composition comprising a suitable pharmaceutical carrier and as an active ingredient an effective serotonin-antagonistic amount of a compound as claimed in any one of claims 1 to 4.

**7.** A method of preparing a pharmaceutical composition, characterized in that a therapeutically effective amount of a compound as defined in any of claims 1 to 4 is intimately mixed with suitable pharmaceutical carriers.

**8.** A compound as claimed in any one of claims 1 to 4 for use as a medicine.

**9.** A compound as claimed in any one of claims 1 to 4 for use as a serotonin-antagonistic medicine.

**10.** A process for preparing a chemical compound as claimed in claims 1, characterized by
a) reacting a piperidine of formula

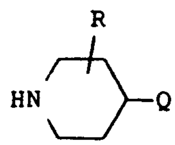

(III),

with a reactive ester of formula

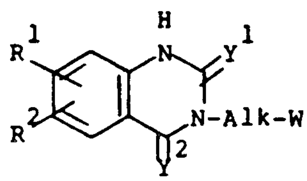

(II),

wherein W represents a reactive leaving group, in a reaction-inert solvent;
b) cyclizing an intermediate of formula

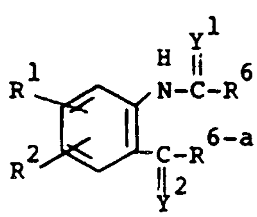

(IV)

wherein $R^6$ and $R^{6\text{-}a}$ each represent a reactive leaving group such as, for example, $C_{1-6}$ alkyloxy, amino and mono- and di($C_{1-6}$ alkyl)amino, with an amine of formula

$D\text{-}NH_2$ (V),

optionally in a reaction-inert solvent, wherein during the reaction of (IV) with (V) the intermediately

formed amide of formula

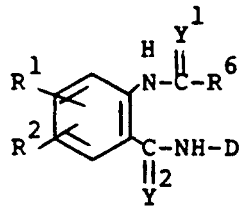

(VI)

may be isolated and subsequently cyclized;
c) cyclizing an isocyanate or isothiocyanate of formula (VII)

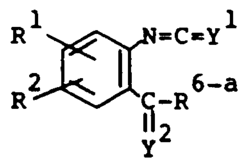

(VII),

with on amine of formula (V), optionally in a reaction-inert solvent;
d) reducing a nitro containing compound of formula

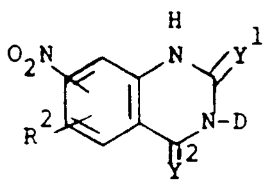

(VIII)

thus preparing a compound of formula

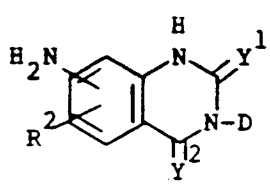

(I-a),

wherein D represents a radical of formula

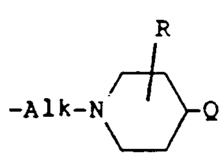

;

or, optionally converting the compounds of formula (I) into each other following art-known groupstransformation procedures, and, if desired, converting the compounds of formula (I) into a therapeutically active non-toxic acid-addition salt form by treatment with an appropriate acid or, conversely, converting the acid-addition salt into the free base form with alkali.

## Revendications

**1.** Composé chimique ayant la formule

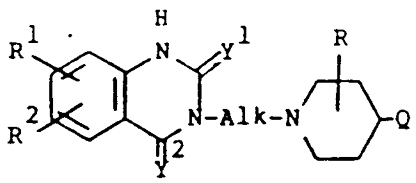

(I)

et sel d'addition de celui-ci avec un acide pharmaceutiquement acceptable, où

| | |
|---|---|
| R | est l'hydrogène ou un groupe alkyle en $C_{1-6}$; |
| $R^1$ | est un groupe amino, mono- et di(alkyl en $C_{1-6}$)amino, alkyl($C_{1-10}$)carbonylamino ou azido; |
| $R^2$ | est l'hydrogène ou un radical halogéno; |
| $Y^1$ et $Y^2$ | sont chacun, indépendamment l'un de l'autre, O ou S; |
| Alk | est un radical alcanediyle en $C_1$-$C_6$; et |
| Q | est le groupe 1 H-indol-3-yle ou un radical de formule |

-X-Ar (a)

où Ar est un groupe aryle; et X est un radical bivalent choisi parmi >C=O; >CHOH; >CH-O-C(=O)-$R_a$; >$CH_2$; >C(O-alkyl)en $C_{1-6}$)$_2$;

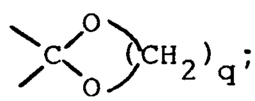

>C=NOH et >C=N-$NH_2$

ledit $R_a$ étant l'hydrogène ou un groupe alkyle en $C_{1-6}$ et ledit q étant l'entier 2 ou 3; et où on désigne par aryle dans la définition de Ar un groupe phényle éventuellement substitué par jusqu'à trois groupes halogéno-, alkyl- en $C_{1-6}$, alkyloxy-en $C_{1-6}$, trifluorométhyle ou amino, ou un groupe thiényle ou pyridinyle.

**2.** Composé chimique selon la revendication 1, dans lequel $R^1$ est un groupe amino, acétylamino ou azido, $R^2$ et R sont tous deux de l'hydrogène et Q est -CO-Ar.

**3.** Composé chimique selon la revendication 1, dans lequel le composé est la 7-amino-3-[4-(4-fluorobenzoyl)-1-pipéridinyl]butyl-2,4(1H,3H)-quinazolinedione.

**4.** Composé chimique selon la revendication 1, dans lequel le composé est la 7-azido-3-[2-[4-(4-fluorobenzoyl) -1-pipéridinyl]éthyl]-2,4(1H,3H)-quinazolinedione.

**5.** Composition pharmaceutique comprenant un support pharmaceutique approprié et en tant qu'ingrédient actif une quantité thérapeutiquement efficace d'un composé selon l'une quelconque des revendications 1 à 4.

**6.** Composition antagoniste de la sérotonine comprenant une support pharmaceutique approprié et en tant qu'ingrédient actif une quantité efficace comme antagoniste de la sérotonine d'un composé selon l'une quelconque des revendications 1 à 4.

**7.** Méthode pour la préparation d'une composition pharmaceutique, caractérisée en ce qu'on mélange intimement avec des supports pharmaceutiques appropriés une quantité thérapeutiquement efficace d'un composé tel que défini dans l'une quelconque des revendications 1 à 4.

**8.** Composé selon l'une quelconque des revendications 1 à 4 pour une utilisation comme médicament.

**9.** Composé selon l'une quelconque des revendications 1 à 4 pour une utilisation comme médicament antagoniste de la sérotonine.

**10.** Procédé pour la préparation d'un composé chimique selon la revendication 1, caractérisé en ce qu'il comprend

a) la réaction d'une pipéridine de formule

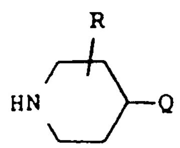

(III),

avec un ester réactif de formule

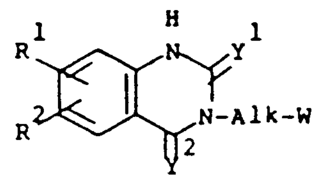

(II),

où W représente un groupe libérable réactif, dans un solvant inerte vis-à-vis de la réaction;

b) la cyclisation d'un intermédiaire de formule

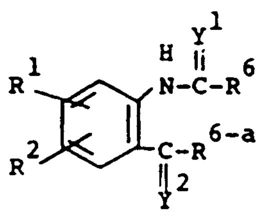

(IV)

où $R^6$ et $R^{6\text{-}a}$ représentent chacun un groupe séparable réactif tel que, par exemple, alkyloxy en $C_{1-6}$, amino et mono- et di(alkyl en $C_{1-6}$)amino, avec une amine de formule

$D\text{-}NH_2$ (V),

en option dans un solvant inerte vis-a-vis de la réaction, tandis que pendant la réaction de (IV) avec (V) l'amide formé à titre d'intermédiaire de formule

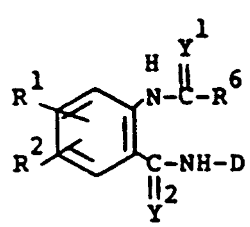

(VI)

peut être isolé et cyclisé ultérieurement;

c) la cyclisation d'un isocyanate ou d'un isothiocyanate de formule (VII)

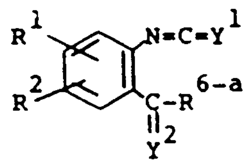
(VII),

avec une amine de formule (V), en option dans un solvant inerte vis-à-vis de la réaction;
d) la réduction d'un composé contenant un groupe nitro de formule

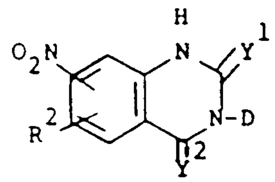
(VIII)

pour préparer un composé de formule

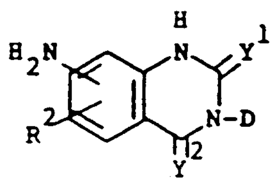
(I-a),

où D représente un radical de formule

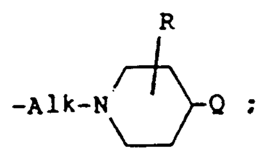
;

ou, en option, la conversion des composés de formule (I) l'un en l'autre suivant des techniques de transformation de groupes connues de l'homme du métier, et, si on le désire, la conversion des composés de formule (I) en une forme de sel d'addition avec un acide non-toxique thérapeutiquement actif par traitement avec un acide approprié, ou au contraire la conversion du sel d' addition avec un acide en la forme de base libre avec un alcali.

## Patentansprüche

**1.** Chemische Verbindung mit der Formel

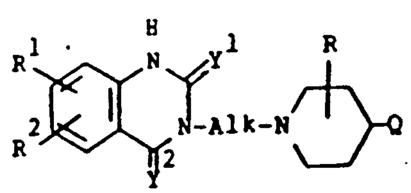
(I)

und ein pharmazeutisch annehmbares Säureadditionssalz hievon, worin

| | |
|---|---|
| R | für Wasserstoff oder $C_{1-6}$Alkyl steht; |
| $R^1$ | Amino, Mono- und Di-($C_{1-6}$alkyl)amino, $C_{1-10}$Alkylcarbonylamino oder eine Azidogruppe darstellt; |
| $R^2$ | für Wasserstoff oder Halogen steht; |
| $Y^1$ und $Y^2$ | jeweils unabhängig voneinander O oder S bedeuten; |
| Alk | für einen $C_1$-$C_6$Alkandiylrest steht; und |
| Q | für 1H-Indol-3-yl oder einen Rest der Formel |

-X-AR (a)

steht, worin Ar Aryl bedeutet; und X einen zweiwertigen ist darstellt, ausgewählt aus der aus $>$C=O; $>$CHOH; $>$CH-O-C(=O)-$R_a$; $>$$CH_2$; $>$C(O-$C_{1-6}$alkyl)$_2$;

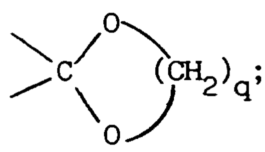

$>$C=NOH und $>$C=N-$NH_2$

bestehenden Gruppe, wobei der $R_a$ Wasserstoff oder $C_{1-6}$Alkyl darstellt und q die ganze Zahl 2 oder 3 bedeutet;

worin Aryl, wie es in der Definition von Ar verwendet wird, Phenyl, das gegebenenfalls durch bis zu drei Halogen-, $C_{1-6}$Alkyl-, $C_{1-6}$Alkyloxy-, Trifluormethyl- oder Aminogruppen substituiert ist, oder Thienyl oder Pyridinyl bedeutet.

**2.** Chemische Verbindung nach Anspruch 1, worin

| | |
|---|---|
| $R^1$ | für Amino, Acetylamino oder Azido steht, |
| $R^2$ | und R beide Wasserstoff bedeuten und |
| Q | für -CO-Ar steht. |

**3.** Chemische Verbindung nach Anspruch 1, wobei es sich um die Verbindung 7-Amino-3-[4-(4-fluorbenzoyl)-1-piperidinyl]butyl-2,4(1H,3H)chinazolindion handelt.

**4.** Chemische Verbindung nach Anspruch 1, wobei es sich um die Verbindung 7-Azido-3-[2-[4-(4-fluorbenzoyl)-1-piperidinyl)ethyl]-2,4-(1,3H)chinazolindion handelt.

**5.** Pharmazeutische Zusammensetzung, umfassend einen geeigneten pharmazeutischen Träger und als einen wirksamen Bestandteil eine therapeutisch wirksame Menge einer Verbindung nach eine der Ansprüche 1 bis 4.

**6.** Serotonin-antagonistische Zusammensetzung, umfassend einen geeigneten pharmazeutischen Träger und als einen wirksamen Bestandteil eine wirksame Serotonin-antagonistische Menge einer Verbindung nach einem der Ansprüche 1 bis 4.

**7.** Verfahren zur Herstellung einer pharmazeutischen Zusammensetzung, dadurch gekennzeichnet, daß eine therapeutisch wirksame Menge einer Verbindung nach einem der Ansprüche 1 bis 4 innig mit geeigneten pharmazeutischen Trägern vermischt wird.

**8.** Verbindung nach einem der Ansprüche 1 bis 4 zur Anwendung als eine Medizin.

**9.** Verbindung nach einem der Ansprüche 1 bis 4 zur Anwendung als eine Serotonin-antagonistische Medizin.

**10.** Verfahren zur Herstellung einer Verbindung nach Anspruch 1, gekennzeichnet durch:

a) Umsetzen eines Piperidins der Formel

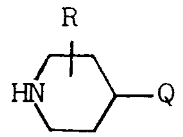 (III)

mit einem reaktionsfähigen Ester der Formel

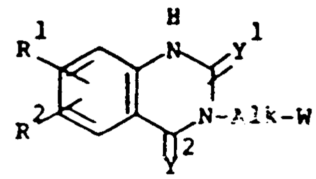 (II),

worin W eine reaktionsfähige Leaving-Gruppe darstellt, in einem reaktionsinerten Lösungsmittel;
b) Cyclisieren eines Zwischenproduktes der Formel

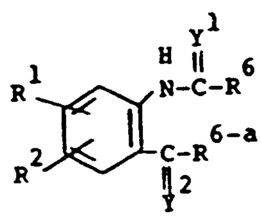 (IV),

worin $R^6$ und $R^{6\text{-}a}$ jeweils eine reaktionsfähige Leaving-Gruppe darstellen, wie z.B. $C_{1-6}$Alkyloxy, Amino und Mono- und Di($C_{1-6}$alkyl)amino, mit einem Amin der Formel

$D\text{-}NH_2$ (V),

gegebenenfalls in einem reaktionsinerten Lösungsmittel, wobei während der Umsetzung von (IV) mit (V) das intermediär gebildete Amid der Formel

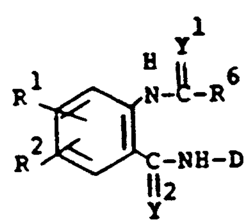 (VI)

isoliert und anschließend cyclisiert werden kann;

c) Cyclisieren eines Isocyanats oder Isothiocyanats der Formel (VII)

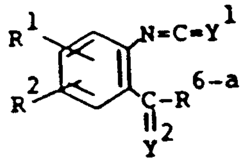

(VII)

mit einem Amin der Formel (V), gegebenenfalls in einem reaktionsinerten Lösungsmittel;
d) Reduzieren einer eine Nitrogruppe enthaltenden Verbindung der Formel

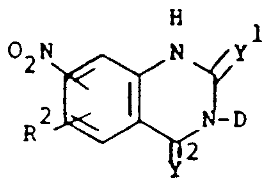

(VIII)

unter Ausbildung einer Verbindung der Formel

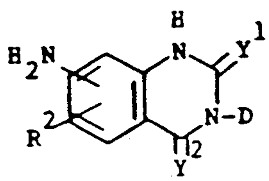

(I-a),

worin D einen Rest der Formel

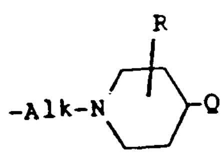

darstellt; oder gewünschtenfalls Überführen der Verbindungen der Formel (I) ineinander nach bekannten Gruppentransformationsverfahren, und gewünschtenfalls Umwandeln der Verbindungen der Formel (I) in eine therapeutisch wirksame nicht-toxische Säureadditionssalzform durch Behandlung mit einer entsprechenden Säure, oder umgekehrt Umwandeln des Säureadditionssalzes mit Alkali in die freie Basenform.